# EUROPEAN PATENT APPLICATION

(11) **EP 0 888 760 A1**
(43) Date of publication of application: **07.01.1999**
(21) Application number: 98610015.4
(22) Date of filing: 03.06.1998
(51) Int. Cl.: A61F 5/445

(54) **An ostomy appliance**

(30) Priority: 04.07.1997 DK 80297
(71) Applicant: COLOPLAST A/S, 3050 Humlebaek (DK)
(72) Inventor: Nielsen, Inger Mann, 2000 Frederiksberg (DK); Olsen, Eskil Höjland, 2930 Klampenborg (DK); Leisner, Henrik, 2820 Gentofte (DK)
(74) Representative: Hegner, Anette

(57) **Abstract**

The ostomy appliance comprises a body side member with a stoma receiving opening having a non-circular contour, e.g. elongated, oval, elliptical or irregular, which in many cases makes it unnecessary for the user to adapt the stoma receiviny opening in the body side member to his or her stoma.

Furthermore, the edge delimiting the stoma receiving opening in the body side member can have a lining consisting of a plastically mouldable adhesive substance, which the user can mould or spread with a finger in order to obtain an oven more close or tight fit to the stoma.

The appliance can be of the one-piece type, where the collection bag is integral with the body side member, or of the two-piece type where the collection bag can be exchanged separately leaving the body side member in place on the skin of the user for use with the next collection bag.

## Description

### FIELD OF THE INVENTION

The present invention relates to an ostomy appliance.

### BACKGROUND OF THE INVENTION

In connection with surgery for a number of diseases in the gastro-intestinal tract such as a colostomy, an ileostomy or a urostomy, a consequence is, in many cases, that the patient is left with an abdominal stoma. In such cases or in connection with a fistula the patient will have to rely on an external ostomy appliance to collect the bodily waste material emerging from such opening called a stoma.

Ostomy appliances are well known. Such appliances may be two-piece or one-piece appliances. In both types of appliances, a collection bag is attached to the user's abdomen in the region of the stoma for receiving waste material or exudates from the stoma.. With two-piece appliances an adhesive faceplate or body side ember is first attached to the user's abdomen, and a receiving member or bag is then attached to the body side ostomy member for receiving waste material from the stoma. In one-piece appliances a collection bag has an integrated adhesive faceplate for attaching the bag to the skin of the user.

When using one-piece appliances, the whole appliance, including the adhesive wafer or pad securing the appliance to the skin is removed and replaced by a fresh appliance. When using two-piece appliances, the body side ostomy member is often left in place for several days, and only the receiving member or bag is replaced.

The service time of the body side ostomy member, i.e. the time the body side ostomy member is in use before it is changed, depends on the amount and the aggressiveness of the exudates from the stoma and of the tightness of the sealing between the stoma and the body side ostomy member.

The stoma often protrudes from the surface of the skin immediately around the stoma, and when the faceplate is attached to the peristomal skin of the user, such protruding or prolapsed stomas are received in an opening in the faceplate. Known ostomy appliances are supplied from the manufacturer with a relatively small stoma receiving opening intended for use by users having a stoma having an irregular contour. When the user takes a fresh faceplate, he or she cuts the relatively small stoma receiving opening with a pair of scissors or a knife to fit around the stoma. This time consuming procedure of fitting the stoma receiving opening to the user's individual stoma must be done with every fresh faceplate.

It is the object of the invention to provide an ostomy appliance that can be applied directly by the users without having to individually adapt the relatively small stoma receiving opening to the user's stoma by cutting.

### SUMMARY OF THE INVENTION

With an ostomy appliance according to the invention the stoma receiving opening in the body side member has a non-circular contour, e.g. elongated, oval, elliptical or irregular, which in many cases makes it unnecessary for the user to adapt, by cutting, the stoma receiving opening in the body side member to his or her stoma.

Furthermore, the edge delimiting the stoma receiving opening in the body side member can have a lining consisting of a plastically mouldable adhesive substance, which the user can mould or spread with a finger in order to obtain an even more close or tight fit to the stoma.

The appliance can be of the one-piece type, where the collection bag is integral with the body side member, or of the two-piece type where the collection bag can be exchanged separately leaving the body side member in place on the skin of the user for use with the next collection bag.

### BRIEF DESCRIPTION OF THE DRAWINGS

Fig.1 shows schematically a body side member according to the invention, and
Fig. 2 shows schematically the body side member of fig. 1 and a collection bag in sectional view.

### DETAILED DESCRIPTION OF THE INVENTION

A body side member 10 is generally disc shaped with a generally circular outer contour. The body side member 10 has a proximal side 12 with a medical grade adhesive 13 for attaching the body side member 10 to the skin of a user. The body side member 10 has a central opening 14 called a body side member opening which is intended to be placed around or receive the stoma of the user, when the body side member is being attached to the peristomal skin of the user by means of the adhesive 13 oil the proximal side. The medical grade adhesive 13 secures the body side member 10 to the peristomal skin. A variety of such adhesives are known in the art and may be used here, one such formulation being disclosed, for example in the patents DK 147 035 and US 4 551 490.

The edge of the body side member opening 14 has a contour which in this embodiment consists of two half circular arcs interconnected by two linear segments, which together form a closed contour of the body side member opening 14. Such a contour will give a good fit to many irregular stomas. The contour of the body side member opening may of course have other shapes also deviating from the known circular shape in order to give better fit to the stomas of many users, and in particular to irregular stomas or loop stomas. Examples of such contours are elongated, oval, elliptical or irregular shapes.

In the area at the edge of the body side member opening 14 there is a soft, plastically mouldable adhesive and sealing substance 16. When the body side member 10 has been attached to the skin of a user, the user can use a finger to spread or mould this substance around the stoma, so that the substance will give a perfectly tight sealing fit to the stoma. The plastically mouldable adhesive substance 16 may be composed of a hypoallergenic, soft, easy-deformable, non-memory putty like adhesive material and is preferably a hydrocolloid based adhesive or a hydrogel.

The soft, plastically mouldable adhesive and sealing substance 16 may have a composition allowing it to cure or harden after being applied to the peristomal skin of the user and after having been shaped or moulded. Such hardening should render the substance soft elastic instead of initially being plastically mouldable or spreadable, whereby the hardened sealing substance will be able to follow possible movements of the skin and of the stoma. The hardening process can e.g. be initiated by absorption of water, whereby the composition will form cross links.

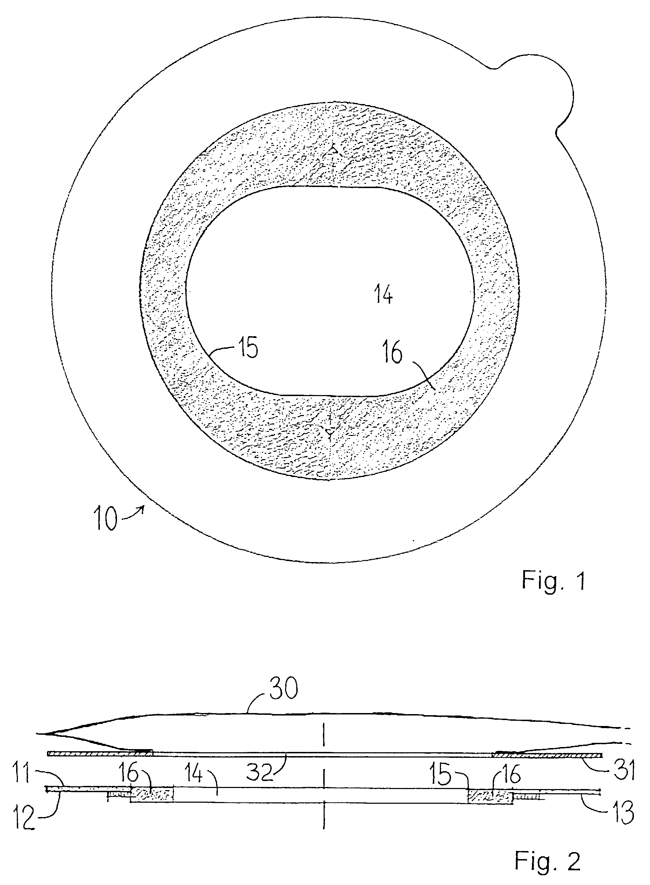

In figure 2 is also shown a collection bag 30 with a flange 31 mounted thereon. The collection bag 30 and the flange 31 have an opening 32 giving access to the interior of the collection bag 30. The collection bag 30 and the flange 31 can be attached to the distal side 11 of the body side member 10 with the bag opening 32 in alignment with the body side member opening 14. In the shown embodiment there is a layer of adhesive on one or both of the contacting faces for securing the collection bag 30 to the body side member 10. The adhesive can be a virtually permanent type of adhesive not intended for being separated, whereby the appliance in figure 2 is a one-piece ostomy appliance.

Alternatively, the adhesive can be of a type permitting the flange 31 to be separated from the body side member 10, or the co-operating faces can each have a respective part of a releasable mechanical coupling such as the ostomy coupling shown in EP 572 378, whereby the appliance is a two-piece ostomy appliance.

## Claims

1. An ostomy appliance comprising
a generally disc shaped body side member having a proximal side and a distal side, said proximal side having an adhesive for securing said body side member to a user's skin, said body side member having a body side member opening therein for receiving a stoma of said user when said body side member is secured to said user's skin, said distal side having first securing means for securing a collection bag thereto, said collection bag having a bag opening therein and second securing means for securing said collection bag to said body side member in cooperation with said first securing means and with said bag opening in alignment with said body side member opening so as to receive bodily excretions from said stoma,
wherein said body side member opening is delimited by an edge having a non-circular contour.

2. An ostomy appliance according to claim 1 wherein said non-circular contour is elongated.

3. An ostomy appliance according to claim 1 wherein said non-circular contour is oval.

4. An ostomy appliance according to claim 1 wherein said non-circular contour is elliptical

5. An ostomy appliance according to claim 1 having a plastically mouldable adhesive substance at saicl edge of said bag opening to permit moulding of said plastically mouldable adhesive substance so as to obtain a close fit to said stoma.

6. An ostomy appliance according to claim 5 wherein said plastically mouldable adhesive substance is cohesive to permit complete removal thereof together with said collection bag from the skin of said user when removing said collection bag from said user's skin.

7. An ostomy appliance according to claim 5 wherein said plastically mouldable adhesive substance is a hardenable substance.

8. An ostomy appliance according to claim 1 wherein said first securing means and said second securing means are adapted to co-operate by means of adhesive.

9. An ostomy appliance according to claim 1 wherein said first securing means and said second securing means for securing a collection bag to said body side member include selectively releasable mechanical coupling means.

10. An ostomy appliance according to claim 1 including a collection bag having a bag opening therein, said collection bag being secured to said body side member with said bag opening in alignment with said body side member opening so as to receive bodily excretions from said stoma.
